(19) Europäisches Patentamt

European Patent Office

Office européen des brevets

(11) **EP 2 778 667 A1**

(12) **EUROPEAN PATENT APPLICATION**

(43) Date of publication:
**17.09.2014 Bulletin 2014/38**

(51) Int Cl.:
***G01N 27/12*** *(2006.01)*    ***G01N 27/27*** *(2006.01)*
***G01N 33/00*** *(2006.01)*

(21) Application number: **13001268.5**

(22) Date of filing: **13.03.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Sensirion AG**
**8712 Stäfa (CH)**

(72) Inventors:
• **Lechner, Moritz**
 **8713 Uerikon (CH)**
• **Bürgi, Lukas**
 **8049 Zürich (CH)**

(74) Representative: **Sutter, Kurt**
**E. Blum & Co. AG**
**Vorderberg 11**
**8044 Zürich (CH)**

(54) **Multi-temperature CMOS gas sensor**

(57)    The gas sensor comprises a membrane (16) with several sensing locations (2a - 4c) arranged thereon. At each sensing location (2a - 4c) a sensing material (20) is provided that changes its resistivity in the presence of gaseous analytes. Electrodes (19) are located on the membrane (16) in electrical contact with the sensing material (20) in order to measure a parameter indicative of the conductance of the sensing material (20). The sensing locations (2a - 4c) are heated by a heater assembly (6, 8) that is structured to generate different temperatures at the sensing locations (2a - 4c), which allows to carry out measurements for different temperatures and to therefrom gain a better understanding of the analytes.

Fig. 1

**Description**

Technical Field

The invention relates to gas sensor for detecting several analytes. The gas sensor has a substrate and sensing material and electrodes arranged on the substrate. The sensing material is selected to change its resistance in the presence of the analytes. The electrodes are in electrical contact with the sensing material. Further, a heater assembly is provided for heating the sensing material. The invention also relates to a method for distinguishing several analytes in a gas by using this type of gas sensor.

Background Art

A sensor of this type is described in GB 2464016. It uses a metal oxide as a sensing material that changes its electrical conductance depending on the composition of the gas that it is exposed to. The sensing material is heated to a suitable operating temperature, typically in the range of 300°C - 600°C. The sensing material is arranged on a membrane for thermal insulation and thermally coupled to a heater.
Wang et al. in "metal oxide gas sensors: sensitivity and influencing factors", Sensors 2010, 10, 2088 - 2106 describe that the sensitivity of metal oxide gas sensors is a function of operating temperature and that the maximum sensitivity of the sensing material varies between different analytes.

Disclosure of the Invention

It is an object of the present invention to provide a gas sensor that allows to distinguish between several analytes quickly.
This object is achieved by the gas sensor of claim 1. Accordingly, the gas sensor for detecting several analytes comprises

- a substrate: the substrate can, for example, be a semiconductor chip having an opening or recess spanned by a membrane;
- a sensing material, arranged on said substrate, changing its resistivity in the presence of said analytes: typical sensing materials are, for example, doped or undoped tin oxide or tungsten oxide;
- electrodes arranged on said substrate in electrical contact with said sensing material: these electrodes are used to measure a parameter indicative of the conductivity of the sensing material;
- a heater assembly for heating said sensing material: this heater allows to heat the sensing material to its operating temperatures.

According to the invention the heater assembly is structured and adapted to generate different temperatures at at least some of the sensing locations. In other words the heater assembly allows, at the same time, to heat the sensing locations, or at least some of them, to differing temperatures. Hence, the sensor is based on the idea to provide several sensing locations on a single sensor and to heat these sensing locations to different temperatures. This allows to distinguish between different analytes in a series of substantially concurrent measurements, without the need to vary the temperature of the measurement locations between measurements.
Advantageously, and as mentioned, the substrate comprises a semiconductor chip having an opening or recess formed therein. The substrate further comprises a membrane extending over said opening or recess. The sensing locations are arranged on the membrane for thermal insulation.
In a particularly advantageous embodiment, the sensing locations are separated by spacer regions. The average thermal conductivity of each of the sensing locations is at least twice as high as the average thermal conductivity of at least some of the spacer regions. In other words, at least some of the spacer regions have much lower thermal conductivity than the sensing locations. This allows to maintain a constant, homogeneous temperature distribution in the sensing locations, while the temperature is changing over the spacer regions having low thermal conductivity. In this context the term "thermal conductivity" describes the in-plane thermal conductance of the substrate or membrane.
The difference in thermal conductance between the sensing locations and the spacer regions can, for example, be achieved by structuring at least one metal layer, in particular a metal layer in the membrane if the device contains a membrane as mentioned above. In particular, the metal layer(s) can be structured such that 75% of the area of each of the sensing locations contains at least one metal layer (i.e. the metal layer(s) extends over at least 75% of the area of each sensing location) and that no more than 25% of the area of at least some of the spacer regions contains any metal layer (i.e. at least 75% of the area of these spacer regions does not contain any metal layer and the metal layers do not extend over more than 25% of the area of these spacer regions). The metal layer(s) that can be structured in this manner include, in particular, the metal layer of the heating assembly and/or the metal layer of the electrodes.

In an advantageous embodiment the heater assembly comprises a heater metal structure and a current or voltage source generating a current in the heater metal structure. The heater metal structure is structured such that it generates different average heating power (per sensing location) for at least some of said sensing locations.

Advantageously, the heater assembly is structured and adapted to generate, at the same time, temperatures differing by more than 20°C, in particular by more than 50°C, at at least some of said sensing locations in order to obtain measurements at a wide temperature range in a short time.

The invention also relates to a method for distinguishing several analytes in a gas by using the gas sensor described above.

Advantageously, such a method comprises the steps of:

- Heating at least some of said sensing locations to different temperatures. In other words these sensing locations are heated differently, such that the sensing material is operated at different temperatures.
- Measuring, for each sensing location, a parameter indicative of the conductance of said sensing material at said sensing location.
- Combining the measured parameters for distinguishing said analyte. Hence, the conductivity parameters measured at sensing locations at different temperatures are combined to gain an understanding as to which analytes are present and, optionally, to determine the concentrations of these analytes.

The gas sensor above allows to pack a rich gas measurement into a small device, and therefore the present invention is particularly suited for mobile applications. Therefore, the invention also relates to a mobile device, in particular a mobile phone, a portable computer or a tablet computer, comprising the gas sensor.

Brief Description of the Drawings

The invention will be better understood and objects other than those set forth above will become apparent from the following detailed description thereof. Such description makes reference to the annexed drawings, wherein:

Fig. 1 shows a view of a first embodiment of a gas sensor,
Fig. 2 shows a top view of the gas sensor of Fig. 1,
Fig. 3 shows a sectional view of the gas sensor of Fig. 1,
Fig. 4 shows a top view of the heater metal layer of a second embodiment of the gas sensor, and
Fig. 5 a mobile phone comprising a gas sensor.

Note: The drawings are not to scale.

Modes for Carrying Out the Invention

Definitions:

Terms indicating a vertical direction or arrangement, such as "top", "bottom", "above" or "below" relate to a frame of reference where the batch of material layers forming the membrane are arranged on top, i.e. above, the substrate. In other words, the substrate is arranged, by definition, below the material layers and the membrane is located on top of the opening extending through the substrate.

The term "lateral" and "in-plane" are used to describe directions parallel to the top and bottom surfaces of the substrate.

The device:

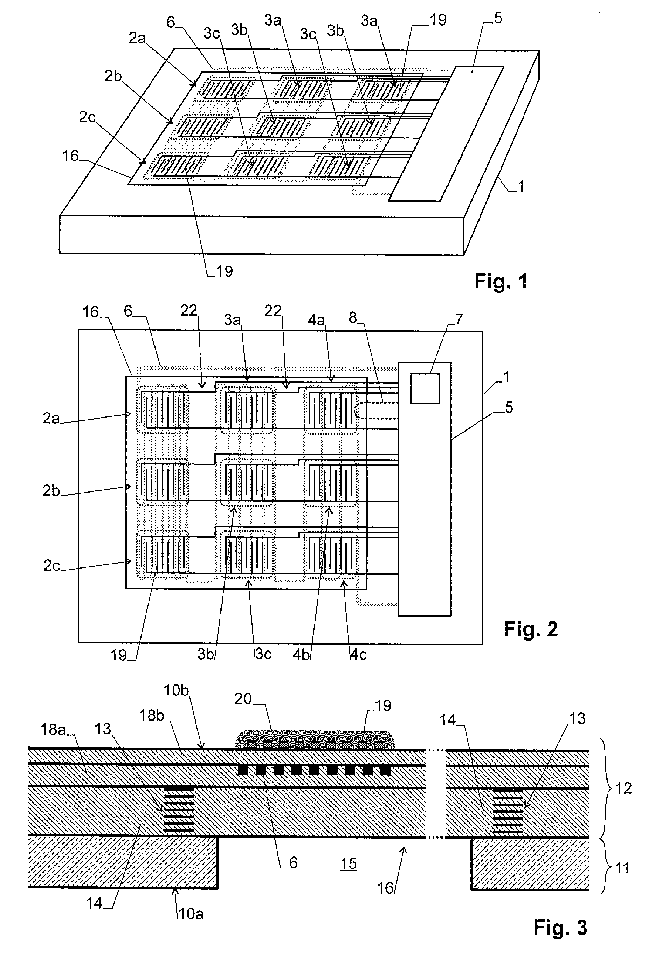

Figs. 1 - 3 show a gas sensor for detecting several gaseous analytes in a gaseous carrier. It comprises a substrate 1. At least one sensing material, whose electrical properties depend on the concentration of the analyte, is applied to substrate 1 in a plurality of sensing locations 2a, 2b, 2c, 3a, 3b, 3c, 4a, 4b, 4c. The sensing material can be applied as a large patch continuously covering all sensing locations and the areas between them, or, advantageously, it can consist of a plurality of smaller patches, each patch corresponding to one sensing location. It can e.g. be applied as a granular layer of tin oxide, tungsten oxide or of another material whose electrical resistance depends on the presence and concentration of the analytes. The various sensing locations 2a - 4c can be covered by different compositions of sensing material.

A gas sensor having a metal oxide sensing material and electrodes for measuring the conductance of such sensing material is e.g. described in GB 2464016 or in WO 95/19563.

At each sensing location the sensing material is in electrical contact with at least a pair of interdigitated electrodes 19, e.g. of platinum, which are connected to a control unit 5. Control unit 5 can be implemented as CMOS circuitry integrated on semiconductor substrate 1. Hence, in an advantageous embodiment, the gas sensor comprises CMOS circuitry integrated on semiconductor substrate 1.

The sensor device further contains a heater assembly comprising a heater metal structure 6, which is shown as a dotted gray region in Figs. 1 and 2. The heater assembly further comprises a current or voltage source 7, which forms part of control unit 5. Current or voltage source 7 is adapted to generate a heating current in the metal structure 6. Metal structure 6 is structured to form metal leads carrying said current at each of the sensing locations 2a - 4c in order to heat them to temperatures of at least 150°C, typically to at least 300°C for at least some of the sensing locations.

The device can also be equipped with temperature sensors for measuring the temperature at the sensing locations 2a - 4c. One such temperature sensor is schematically shown under reference number 8 in Fig. 2. This temperature sensor 8 can e.g. be a platinum temperature sensor formed by a platinum conductor extending over membrane 16, or it can be a thermopile.

The temperature signals from the temperature sensors 8 can be used to regulate the temperature at the sensing locations 2a - 4c (if the heater assembly comprises an individually controllable heater for each sensing location, see "further embodiments" below), or it can be used to correct the measured data for the difference between target and actual temperatures.

As can be seen from the sectional view of Fig. 3, substrate 1 comprises a bottom surface 10a and a top surface 10b. Bottom surface 10a is formed by a semiconductor chip 11. A batch 12 of material layers is applied at top surface 10b and typically comprises a plurality of structured dielectric layers and a plurality of structured metal layers.

A bottommost part 13 of the various metal layers is typically of aluminum (or AlCu or copper) and forms interconnects of the CMOS circuitry of control unit 5. In Fig. 3, the metal layers 13 are only shown schematically. They are separated by dielectric layers, typically silicon oxide layers, which are generally denoted by reference number 14.

Parts of the layers of batch 12 extend over an opening or recess 15 in semiconductor chip 11 and form a membrane 16. Membrane 16 can have circular or rectangular shape or any other suitable shape.

Advantageously, and in order to reduce the thermal conductance of membrane 16, none of the metal layers 13 extends into membrane 16.

Batch 12 can further comprise a layer of SiN (not shown) under tensile stress, which extends at least over membrane 16 and is anchored laterally outside membrane 16. The tensile stress in this layer can be at least sufficiently large to exceed the compressive stress in the rest of membrane 16, which leads to a total tensile stress in the membrane. As described in US 7 154 372, such a tensile layer can be used to prevent the membrane from buckling.

Heater metal layer 6 is e.g. a structured tungsten layer, which is located in a silicon oxide layer (or other dielectric layer) 18a on membrane 16. As seen in Figs. 1 and 2, heater metal layer 6 can e.g. form meandering metal leads at each sensing location 2a - 4c.

A dielectric layer 18b, e.g. silicon oxide, is arranged on top of heater metal layer 6 and electrically insulates the same from a platinum layer forming the electrodes 19. As mentioned, the electrodes 19 can e.g. form interdigitated electrode pairs at each sensing location 2a - 4c, which electrode pairs are connected to control unit 5 in such a manner that the conductance of the sensing material at each sensing location 2a - 4c can be measured individually.

A protective dielectric layer (not shown) can be applied to cover at least part of the device.

The sensing material, which is denoted by reference number 20 in Fig. 3, is applied to the top of the device.

Temperature control:

As can be seen from Fig. 2, the meandering leads of heater metal layer 6 are located to heat the sensing locations 2a - 4c. However, heater metal layer 6 is structured such that it generates a larger heating power per unit area at some sensing locations as compared to other sensing locations. In the embodiment of Figs. 1 - 3 this is achieved by keeping the width of the leads of heater metal layer 6 constant, but by arranging a larger number (density) of leads beneath the sensing locations 2a - 2c, while locating a smaller number of leads at the sensing locations 3a - 3c and an even smaller number at the sensing locations 4a - 4c. For this reason, the sensing locations 2a - 2c will be heated to a higher temperature than the sensing locations 3a - 3c, while the temperature at the sensing locations 4a - 4c will be even lower than the temperature at the sensing locations 3a - 3c. On the other hand the temperatures at the sensing locations 2a - 2c will be substantially equal, same as the temperatures at the sensing locations 3a - 3c and the temperatures at the sensing locations 4a - 4c.

By structuring the heater arrangement 6, 7 in such a manner that it generates the same temperature at the same time at several sensing locations, several measurements can be taken for the same temperature, e.g. in order to obtain redundant measurements or to carry out measurements with different sensing materials (e.g. differently doped tin oxides) at the same temperature. For example, the sensing locations 2a, 3a, 4a can be covered with a first sensing material, while the sensing locations 2b, 3b, 4b are covered with a second sensing material and the sensing locations 2c, 3c, 4c are covered with a third sensing material.

As it also can be seen from Fig. 2, there are spacer regions 22 located between the sensing locations 2a - 4c. In the spacer regions 22 located between two sensing locations of different temperatures, the various metal layers have a low degree of coverage. Hence and as mentioned above, these spacer regions have a low thermal conductivity and provide for good thermal insulation between the adjacent sensing locations.

Operation:

Control unit 5 is structured and adapted to carry out a measurement at each sensing location and to derive a parameter p(i) indicative of the conductance of the sensing material at the sensing location, where i is an index identifying the sensing location. Using these parameters p(i), control unit 5 is able to distinguish several types of analytes.

This can, for example, be understood by referring to Fig. 14 of the publication of Wang et al. mentioned above, which shows, for a tin oxide sensor, a higher sensitivity to ethanol then to chlorobenzene at 200°C while the sensitivity is higher for chlorobenzene at 370°C. Similar examples of temperature and material dependent sensitivities for tin oxide gas sensors have e.g. been shown by J. Watson et al. in Meas. Sci. Technol. 4(1993), 711 - 719, see e.g. Section 6, where it is described that the working temperature of a tin oxide sensor can be chosen to enhance the sensitivity to certain gases by comparison with others.

For each sensing location i, control unit 5 uses the respective electrodes 19 to measure the parameter p(i) indicative of the conductance of the sensing material at said sensing location. Parameter p(i) can e.g. be a measure of the current flowing through sensing location i for a given applied voltage, or of the voltage required to maintain a given current. Parameter p(i) is processed by control unit 5, or by hard- and software external to substrate 1, in order to detect which analytes are present.

In general, if it is assumed that the gas sensor is to measure the concentration c(j) of j = 1 ... K analytes, and it measures the parameters p(i) of i = 1 ... M sensing cells, the concentrations can be calculated from a function F as

$$c(j) = F(j, p(1), \ldots p(M)) \qquad (1)$$

Function F can e.g. be a heuristic, parameterized function with function parameters $f_m$, i.e.

$$c(j) = F(j, p(1), \ldots p(M), f_1, \ldots f_M) \qquad (2)$$

The function parameters $f_1 \ldots f_M$ can be found in a calibration. Such a calibration generally comprises the steps of

- exposing the gas sensor to different environments, with each environment having known concentrations c(j) of the gases j = 1 .. K;
- fitting the function parameters $f_m$ to find function F such that the errors in Eq. (2) are minimized.

It must be noted that this is just one way to determine the function F. The above method can, e.g., be refined by adjusting the function parameters $f_m$ over time, e.g. by means of recalibration, taking into account that the gas sensor may be subject to ageing effects.

Once that the function F is known, control unit 5 is able to distinguish the analytes by means of a measurement of the parameters p(i).

One of the advantages of the present device (over a device that varies the temperature of a sensing location over time in order to take consecutive measurements at different temperatures) is the capability to carry out concurrent measurements at different temperatures, thereby reducing the time required for a measurement. Hence, in an advantageous embodiment, the present method for distinguishing several analytes in a gas comprises the step of concurrently heating at least some of the sensing locations 2a - 4c to different temperatures while measuring, for each sensing location, the parameter indicative of the conductance of the sensing material. The actual conductance measurements can also be carried out concurrently, or they can be carried out consecutively - however, even if they are carried out consecutively, there is no need to change the temperature of the sensing locations between two measurements, which makes the process much faster.

Concurrently heating at least some of the sensing locations 2a - 4c to the different operating temperatures allows to reduce the time for taking a complete measurement at different temperatures to less than 10 s, in particular less than 5 s, even to less than 2 s.

Further embodiments:

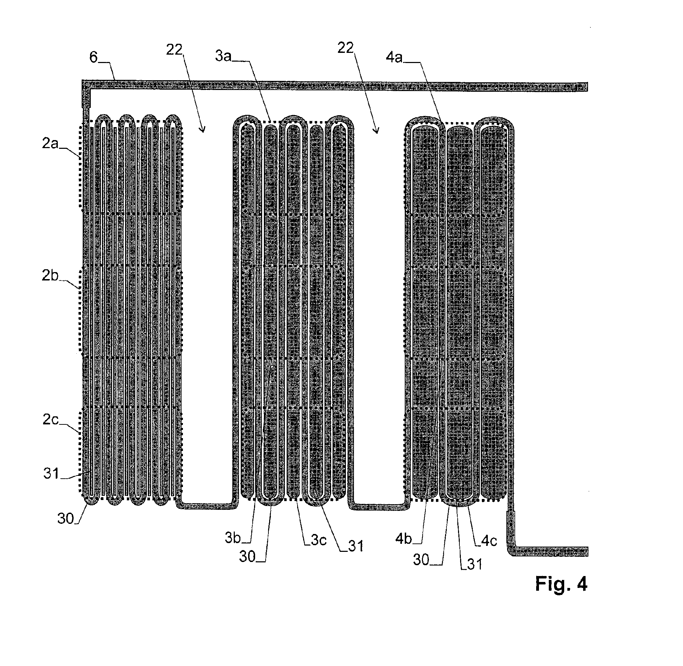

Fig. 4 shows a top view of the heater metal layer of a second embodiment of the gas sensor, where the sensing locations 2a - 4c are denoted by dotted lines. This embodiment differs from the one of Figs. 1 - 3 in that the heater metal layer contains not only the metal leads 30 that carry the heating current, but also additional metal structures 31 between the metal leads 30 that do not carry any substantial current. However, the additional metal structures 31 are only provided at the sensing locations 2a - 4c and, optionally, between sensing locations that are to be at the same temperature. There are no additional metal structures 31 between sensing locations that are to be at different temperatures, i.e. at the spacer regions 22. As mentioned above, this design allows to maintain a constant, homogeneous temperature distribution in the sensing locations 2a - 4c, while the temperature drops over the spacer regions 22.

Alternatively or in addition to the design of Fig. 4, the width of the leads 30 can be varied and they can be wider at the sensing locations that are to be at lower temperatures.

The above designs have three sensing locations for each temperature, for example the three sensing locations 2a, 2b, 2c are at the highest temperature. The number of sensing locations at each temperature can, however, also be smaller, even 1, or larger.

The geometry of the gas sensor can also be varied. For example, the hottest sensing location can be located at the center of membrane 16, with the sensing locations for lower temperatures surrounding it. This design takes into account that it is easier to heat the center of membrane 16 then its peripheral areas.

In the above embodiments, the gas sensor comprises a single membrane 16 and all sensing locations are arranged on said membrane. Alternatively, the gas sensor may comprise several membranes, with each membrane carrying only a subset of the sensing locations.

Further, in the above embodiments, the heater assembly formed a single, meandering conducting lead extending over all heater assemblies. However, alternatively, individual heater units, which may be operated in series, in parallel or independently of each other, may be provided for each sensing location 2a - 4c.

In yet a further embodiment, the membrane can be dispensed with if the gas sensor is otherwise structured to exhibit sufficiently low thermal conductance and capacity at the sensing locations.

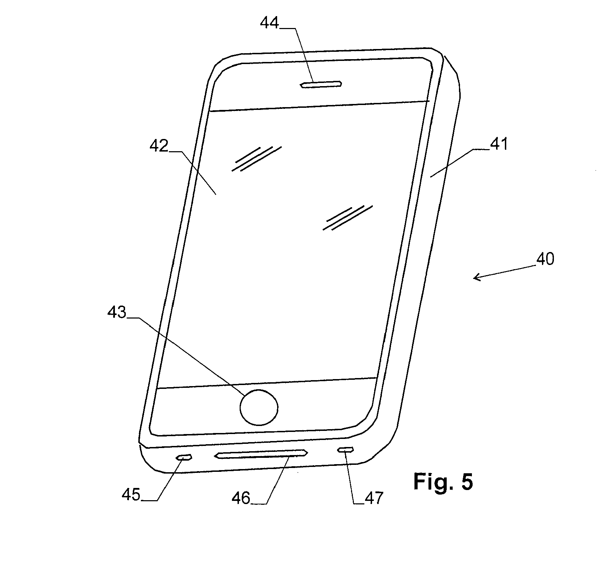

As mentioned, the present invention is particularly suited for being used in a mobile device. Fig. 5 shows such a mobile device, namely a mobile phone 40, having a housing 41, a display 42, user controls 43 as well as a plurality of openings 44 - 47. The gas sensor can be arranged behind any of these openings, advantageously behind an opening 45 or 47 dedicated to the use as a gas sensor inlet.

While there are shown and described presently preferred embodiments of the invention, it is to be distinctly understood that the invention is not limited thereto but may be otherwise variously embodied and practised within the scope of the following claims.

**Claims**

1. A gas sensor for detecting several analytes comprising
a substrate (1),
a sensing material (20) changing its resistivity in the presence of said analytes, wherein said sensing material (20) is arranged on said substrate (1),
electrodes (19) arranged on said substrate (1) in electrical contact with said sensing material (20), and
a heater assembly (6, 7) for heating said sensing material (20),
**characterized in that**
said electrodes (19) are structured to individually measure a parameter indicative of a conductance of said sensing material (20) at several sensing locations (2a - 4c) on said substrate (1) and
said heater assembly (6, 7) is structured and adapted to generate differing temperatures at at least some of said sensing locations (2a - 4c).

2. The gas sensor of claim 1, wherein said substrate (1) comprises
a semiconductor chip (11) having an opening or recess (15) and
a membrane (16) extending over said opening or recess (15),
wherein said sensing locations (2a - 4c) are arranged on said membrane (16).

3. The gas sensor of claim 2 further comprising CMOS circuitry integrated on said semiconductor chip (11).

**4.** The gas sensor of any of the preceding claims, wherein the sensing locations (2a - 4c) are separated by spacer regions (22), wherein an average thermal conductivity of each of said sensing locations (2a - 4c) is at least twice as high as an average thermal conductivity of at least some of said spacer regions (22).

**5.** The gas sensor of claim 4, wherein at least 75% of the area of each of said sensing locations (2a - 4c) contains at least one metal layer, and wherein no more than 25% of the area of at least some of said spacer regions (22) contains a metal layer.

**6.** The gas sensor of any of the preceding claims, wherein said heater assembly (6, 7) comprises a heater metal structure (6) and a current or voltage source (7) generating a current in said heater metal structure (6), wherein said heater metal structure (6) is structured for generating different average heating power per sensing location for at least some of said sensing locations (2a - 4c).

**7.** The gas sensor of any of the preceding claims further comprising a control unit (5) structured and adapted
to measure, by means of said electrodes (19), parameters p(i) indicative of the conductance of said sensing material (20) at said sensing locations i and
to distinguish several analytes from said parameters p(i).

**8.** The gas sensor of any of the preceding claims wherein said sensing material (20) comprises a metal oxide, in particular tin oxide and/or tungsten oxide.

**9.** The gas sensor of any of the preceding claims wherein said heater assembly (6, 7) is structured and adapted to generate the same temperature at the same time at several sensing locations (2a - 4c).

**10.** The gas sensor of any of the preceding claims wherein said heater assembly (6, 7) is structured and adapted to generate, at the same time, temperatures differing by more than 20°C, in particular by more than 50°C, at at least some of said sensing locations (2a - 4c).

**11.** The gas sensor of any of the preceding claims further comprising temperature sensors (8) located at the sensing locations (2a - 4c).

**12.** A mobile device, in particular a mobile phone, a portable computer or a tablet computer, comprising the gas sensor of any of the preceding claims.

**13.** A method for distinguishing several analytes in a gas by using the gas sensor of any of the preceding claims.

**14.** The method of claim 13 comprising the steps of
heating at least some of said sensing locations (2a - 4c) to different temperatures,
measuring, for each sensing location, a parameter indicative of a conductance of said sensing material (20) at said sensing location, and
combining said parameters for distinguishing said analytes.

**15.** The method of claim 14 wherein said at least some sensing locations (2a - 4c) are concurrently heated to different temperatures while measuring, for each sensing location (2a - 4c), the parameter indicative of the conductance of the sensing material (20).

Fig. 1

Fig. 2

Fig. 3

Fig. 4

**Fig. 5**

EP 2 778 667 A1

## EUROPEAN SEARCH REPORT

Application Number

EP 13 00 1268

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | R. E. CAVICCHI ET AL: "Microhotplate Gas Sensor Arrays", PART OF THE SPIE CONFERENCE ON CHEMICAL MICROSENSORS AND APPLICATIONS II BOSTON. MASSACHUSETTS, vol. 3857, 30 September 1999 (1999-09-30), pages 38-49, XP055074738, * pages 38-40 * | 1-15 | INV. G01N27/12 G01N27/27 G01N33/00 |
| X | GRAF M ET AL: "Smart single-chip CMOS microhotplate array for metal-oxide-based gas sensors", TRANSDUCERS, SOLID-STATE SENSORS, ACTUATORS AND MICROSYSTEMS, 12TH INN ATIONAL CONFERENCE ON, 2003, PISCATAWAY, NJ, USA,IEEE, 8 June 2003 (2003-06-08), pages 123-126vol.1, XP032377446, DOI: 10.1109/SENSOR.2003.1215268 ISBN: 978-0-7803-7731-8 * pages 123,124 * | 1-15 | |
| X | GB 2 422 017 A (UNIV WARWICK [GB]) 12 July 2006 (2006-07-12) * page 8, lines 29-33 * | 1-3,8, 13-15 | TECHNICAL FIELDS SEARCHED (IPC)  G01N |
| X | MULLER G ET AL: "A MEMS toolkit for metal-oxide-based gas sensing systems", THIN SOLID FILMS, ELSEVIER-SEQUOIA S.A. LAUSANNE, CH, vol. 436, no. 1, 22 July 2003 (2003-07-22) , pages 34-45, XP004431389, ISSN: 0040-6090, DOI: 10.1016/S0040-6090(03)00523-6 * the whole document * | 1,2, 6-10, 13-15 | |

The present search report has been drawn up for all claims

| Place of search | Date of completion of the search | Examiner |
|---|---|---|
| Munich | 9 August 2013 | Stussi, Elisa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

11

## ANNEX TO THE EUROPEAN SEARCH REPORT
## ON EUROPEAN PATENT APPLICATION NO.

EP 13 00 1268

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2013

| Patent document cited in search report | Publication date | Patent family member(s) | Publication date |
|---|---|---|---|
| GB 2422017 A | 12-07-2006 | GB 2422017 A<br>JP 4936677 B2<br>JP 2006194853 A<br>US 2006154401 A1 | 12-07-2006<br>23-05-2012<br>27-07-2006<br>13-07-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2464016 A
- WO 9519563 A

- US 7154372 B

**Non-patent literature cited in the description**

- **WANG et al.** metal oxide gas sensors: sensitivity and influencing factors. *Sensors,* 2010, vol. 10, 2088-2106

- **J. WATSON et al.** *Meas. Sci. Technol.,* 1993, vol. 4, 711-719